# FASCICULE DE BREVET EUROPEEN

(11) **EP 0 483 005 B1**
(45) Date de publication et mention de la délivrance du brevet: **23.06.1993**
(21) Numéro de dépôt: 91402831.1
(22) Date de dépôt: 23.10.1991
(51) Int. Cl.: A61B 6/00

(54) **Mammographe muni d'un porte-aiguille perfectionné**
Mammographiegerät mit Nadelhalter
Mammography apparatus provided with a needle holder

(30) Priorité: 24.10.1990 FR 9013180
(43) Date de publication de la demande: 29.04.1992
(73) Titulaire: GENERAL ELECTRIC CGR S.A., F-92130 Issy les Moulineaux (FR)
(72) Inventeur: Teubner, Joachim, F-75116 Paris (FR); Dibartolomeo, Jean-Yves, F-75116 Paris (FR); Rouchy, Didier, F-75116 Paris (FR)
(74) Mandataire: Schmit, Christian Norbert Marie

(56) Documents cités:
- EP-A- 0 146 511
- EP-A- 0 288 187
- PROCEEDINGS OF THE INSTITUTION OF MECHANICAL ENGINEERS. vol. 183, no. 15, 1968-1969, LONDON GB pages 281 - 292; DAWSON ET AL.: 'Bio-engineering approach to stereotactic surgery of the brain.'
- PROCEEDINGS OF THE ANNUAL INTERNATIONAL CONFERENCE OF THE IEEE ENGINGEERING IN MEDICINE AND BIOLOGY SOCIETY. vol. 11, 1989, US pages 879 - 880; GUERROUAD ET AL.: 'S.M.O.S. : Stereotaxical Micromanipulator for Ocular Surgery.'

## Description

La présente invention a pour objet un mammographe muni d'un porte-aiguille perfectionné. Elle est plus particulièrement utilisable dans le domaine médical pour préparer des interventions sur des seins de patientes examinées. L'invention vise à faciliter principalement la réalisation d'une biopsie ou un guidage chirurgical au moment d'une ablation d'une tumeur.

Un mammographe comporte normalement un mât auquel est fixé un plateau porte-sein et une pelote mobile. Au moment de l'examen, une patiente pose son sein sur le plateau et la pelote est abaissée de manière à comprimer d'une part, et à maintenir en position fixe d'autre part, le sein sous examen. D'un côté de cet ensemble, généralement au-dessus, est situé un tube à rayons X. De l'autre côté on place un porte-cassette dans lequel on glisse une cassette munie d'un film radiosensible. Le tube à rayons X est en fait fixé à une potence dont le mât est inclinable par rapport au mât du mammographe. En stéréographie, pour une inclinaison d'un côté donné du mât, on prend un cliché radiographique du sein. On bascule ensuite la potence qui porte le tube à rayons X de manière à ce qu'il prenne, de préférence, une inclinaison symétrique de la première par rapport au mât du mammographe. On prend alors un deuxième cliché radiographique. Après développement les clichés peuvent révéler chacun la présence d'une affection particulière que l'on cherche à détecter. En général on cherche à montrer la présence des tumeurs cancéreuses. L'examen stéréographique ainsi mené permet au cours d'une mesure stéréotaxique ultérieure de déterminer dans l'espace, c'est-à-dire par rapport à un repère à trois dimensions attaché soit au porte-cassette soit à la pelote, les coordonnées X,Y,Z des tumeurs détectées dans le sein examiné.

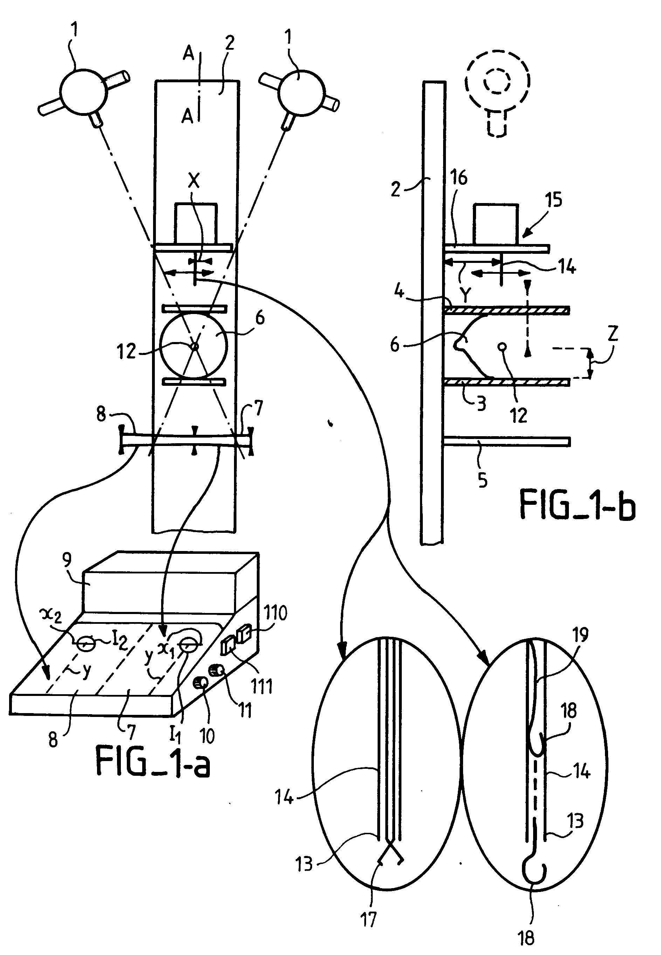

La figure 1a montre, schématiquement, un tel mammographe avec les deux positions possibles du tube 1 à rayons X de part et d'autre du mât 2 du mammographe. La figure 1b est une vue en coupe selon la direction AA de la figure 1a. On distingue accroché au mât 2, le plateau porte-sein 3, la pelote mobile 4, le porte-cassette 5 et le sein 6 sous examen. Lors de l'examen stéréographique on a saisi deux clichés respectivement 7 et 8 que l'on peut visualiser sur un appareil 9 de stéréotaxie.

Cet appareil 9 de stéréotaxie comporte des index, au moins deux index I₁ et I₂ matérialisés ici par des petits ronds entourant des croix. Ces index sont déplaçables sur le plan de visualisation de l'appareil 9 au moyen de boutons de mesure 10 et 11. Des boutons de commande 110 et 111 permettent de choisir de déplacer chacun à leur tour les index I₁ ou I₂. Les boutons 10 et 11 sont dits des boutons de mesure parce que les coordonnées x₁,y et x₂,y des index sont liées aux actions exercées sur ces boutons 10 et 11. On peut ainsi faire pointer, dans chacun des clichés 7 ou 8 respectivement, ces index I₁ ou I₂ sur l'image d'une tumeur 12 contenue dans le sein. On peut alors mesurer les abscisses x₁ et x₂ ainsi que les ordonnées y de ces images. On peut en déduire les coordonnées X,Y,Z de la tumeur. Par exemple
Y = y
X = a x₁+ b x₂
Z = a'x₁ + b'x₂
Dans ces expressions, a,b,a',b' sont des coefficients de changement d'axes, tenant compte de l'inclinaison du rayon principal du tube 1 à rayons X par rapport au mât 2 et de l'éloignement de ce tube 1 des clichés 7 et 8 au moment de la prise des clichés. Le calcul des positions X,Y,Z de la tumeur est de type connu, il est par exemple du type de celui décrit dans la demande de brevet français n° 2 248 535 déposée le 17 octobre 1974 ou encore dans la demande de brevet français n° 2 645 286 déposée le 29 mars 1989.

Pour une intervention, une fois qu'on a repéré la position de la tumeur, on repère la position de l'extrémité 13 de l'aiguille 14 d'un porte-aiguille 15. Si Xₐ, Yₐ et Zₐ sont les positions de cette extrémité, on déplace le porte-aiguille 15 sur un plateau 16 qui le porte de manière à ce que Xₐ soit égal à X et à ce que Yₐ soit égal à Y, Zₐ étant suffisamment différent de Z pour qu'au cours de ce déplacement il n'y ait aucun risque que l'extrémité 13 vienne au contact du sein. La distance Z - Zₐ est dite profondeur d'enfoncement, elle est prédéterminée à l'avance. Par exemple elle vaut 7 cm sur un appareil STEREOTIX de GENERAL ELECTRIC CGR France.

Un appareil de ce type est par exemple décrit dans le document EP-A-0 288 187

L'aiguille 14 associée à un mammographe est une aiguille spéciale : elle est creuse. On peut d'une manière connue y introduire deux outils chirurgicaux, un premier qui est appelé une pince 17, montré sur l'agrandissement de gauche sur la figure 1a, et un autre qui est appelé hameçon 18 qui est montré sur l'agrandissement de droite. La pince sert à réaliser une biopsie, c'est-à-dire un prélèvement d'une partie d'un tissu concerné. L'hameçon sert de guidage chirurgical. Dans les deux cas la manipulation est la même. Elle consiste à provoquer le déplacement du porte-aiguille verticalement selon la longueur d'enfoncement prédéterminée. La pelote 4 est munie d'une ouverture permettant l'enfoncement de l'aiguille. Ceci permet de maintenir le sein comprimé pendant cette opération. Dans ces conditions l'extrémité 13 de l'aiguille vient se placer à l'endroit de la tumeur 12. A ce moment, ou éventuellement aussi au préalable, on introduit soit la pince 17 soit l'hameçon 18 dans l'aiguille. Si on veut effectuer une biopsie on effectue au moyen de la pince 17 un prélèvement, et on retire la pince à l'intérieur de l'aiguille creuse avant de retirer l'aiguille creuse elle même.

Par contre, si on veut montrer à un chirurgien l'endroit où il doit intervenir, on glisse un hameçon dans l'aiguille creuse 14 et on retire l'aiguille creuse en laissant l'hameçon s'accrocher à proximité de la tumeur. L'hameçon 18 est solidaire d'un fil 19 qui apparaît alors à l'extérieur du sein et qui peut montrer au chirurgien jusqu'où il doit aller pour effectuer l'ablation de la tumeur détectée.

L'appareil ainsi décrit avec la manière de s'en servir, présente un inconvénient : au moment de l'intervention chirurgicale, le chirurgien est obligé de suivre le fil qui se termine par l'hameçon. L'orientation de ce fil n'est pas nécessairement la meilleure direction d'intervention possible. Et ceci qu'il s'agisse de la profondeur des tissus incisés ou du caractère inesthétique de la cicatrice post-opératoire. Par ailleurs, il est aussi possible que le chirurgien cisaille le fil et perde l'indication de la position de l'hameçon.

Dans l'invention, on résout ce problème en rendant l'opération d'enfoncement de l'aiguille complètement indépendante de la structure de l'appareil. Ainsi, plutôt que de prévoir un porte-aiguille déplaçable en X et Y et une aiguille enfonçable selon Z, on prévoit un porte-aiguille déplaçable en X,Y et éventuellement Z, mais surtout une aiguille enfonçable selon n'importe quelle direction. Pour résoudre alors automatiquement les problèmes de localisation de la tumeur par rapport à la pointe de l'aiguille, on réalise un porte-aiguille orientable en direction d'un isocentre situé à l'endroit de la tumeur. Ce porte-aiguille est donc prévu pour que l'extrémité de l'aiguille, après un enfoncement de la longueur prédéterminée, soit située à l'endroit de cette tumeur. Dans ces conditions il devient possible, au moment où on effectue la biopsie ou au moment où on place l'hameçon, de choisir une voie d'accès dans le sein qui soit la moins traumatisante possible pour la patiente ou par ailleurs la moins sujette à erreur. Par exemple, les zones les plus centrales du sein peuvent être atteintes à partir de la zone périaréolaire. Dans l'autre cas, la direction du repérage pour la mise en place de l'hameçon peut coïncider avec la direction choisie par le chirurgien pour l'incision. Le fil de repérage sert alors bien mieux de guide pour cette opération. En variante il peut même être préféré que l'hameçon de repérage soit tangent à la zone pathologique et qu'ainsi il ne pénètre pas en son centre.

L'invention a donc pour objet un mammographe comportant
- des moyens pour maintenir un sein d'une patiente entre un dispositif de prise de cliché radiographique et un tube à rayons X ;
- des moyens pour prendre des clichés stéréographiques de ce sein ;
- des moyens de biopsie ou de guidage chirurgical pour intervenir dans ce sein en un endroit déterminé ;
- ces moyens de biopsie ou de guidage chirurgical comportant un porte-aiguille (15) déplaçable (26-35) par rapport au sein ;
- ce porte-aiguille comportant une aiguille (14) dont l'extrémité (13) est déplaçable d'une longueur choisie (D) entre une position en retrait et une position enfoncée ;
- un ensemble (9) stéréotaxique en relation avec ces trois moyens pour calculer la position (X,Y,Z) de l'endroit déterminé de l'intervention ;
   caractérisé en ce qu'il comporte
- des moyens d'orientation (21-25) pour que le porte-aiguille de biopsie soit orientable autour d'un axe qui passe par un isocentre situé à un endroit du sein déterminé, et pour que le porte-aiguille soit incliné par rapport à cet axe de rotation.

L'invention sera mieux comprise à la lecture de la description qui suit et à l'examen des figures qui l'accompagnent. Celles-ci ne sont données qu'à titre indicatif et non limitatif de l'invention.

Les figurent montrent :
- figures 1a et 1b déjà décrites : les éléments essentiels d'un mammographe utilisable dans l'état de la technique et également dans l'invention ;
- figures 2a à 2g : une représentation simplifiée en perspective d'un dispositif permettant une orientation isocentre selon l'invention, et des vues respectivement latérales, en coupe, de dessus et encore en coupe d'un mode préféré de réalisation des moyens d'orientation ;
- figure 3 : un perfectionnement d'un dispositif selon les figures 2 ;
- figure 4 : une réalisation simplifiée de l'invention.

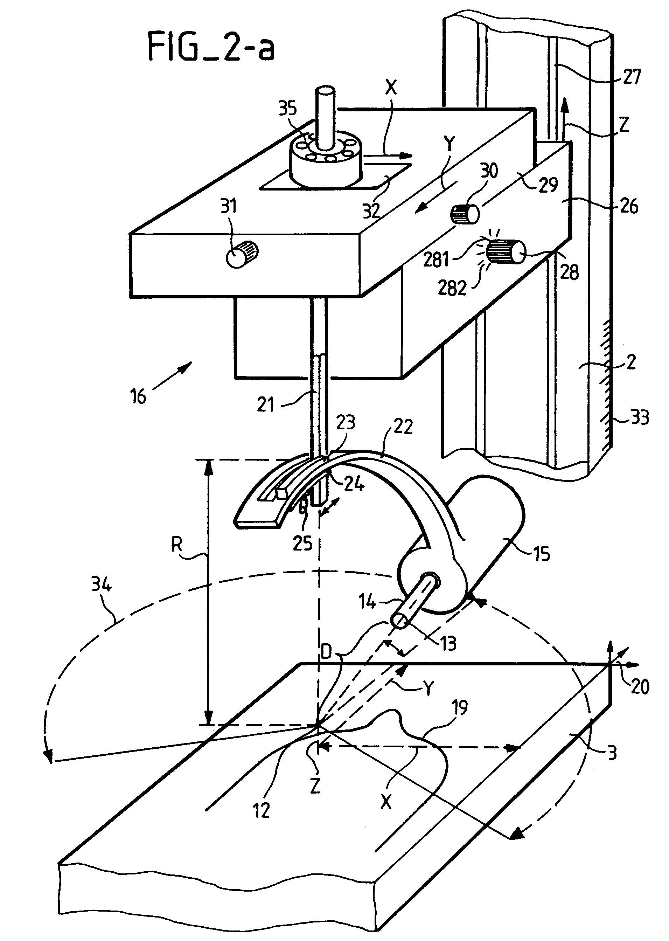

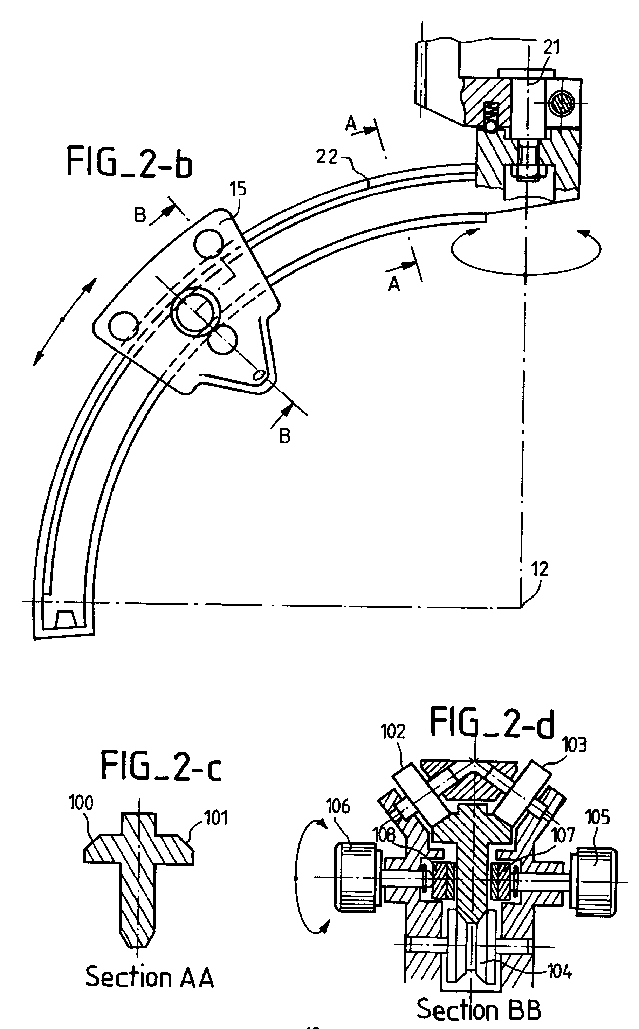

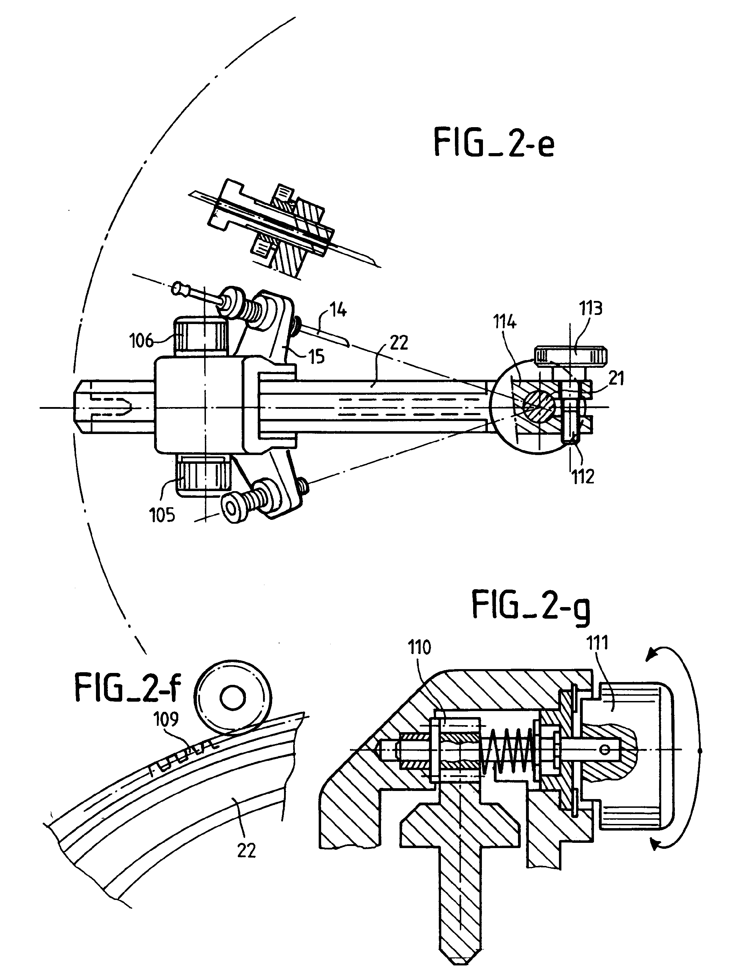

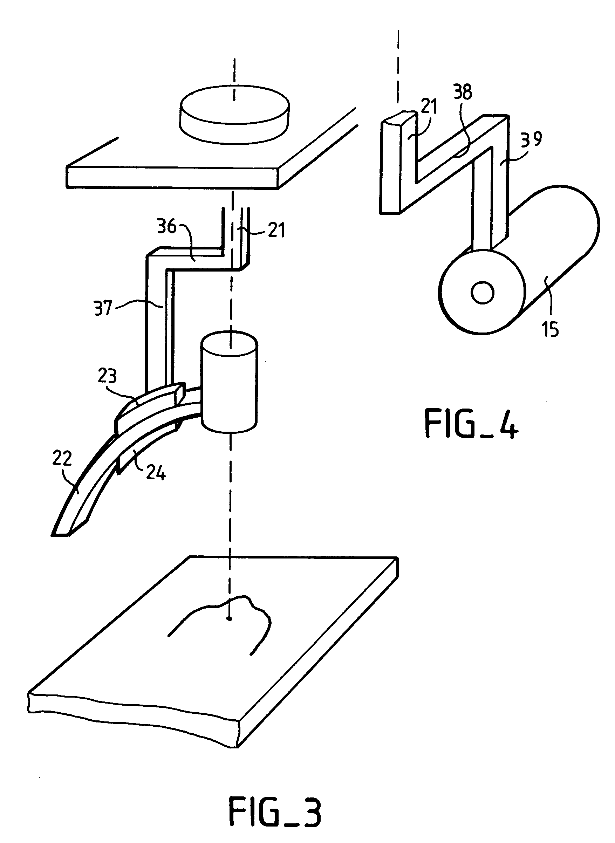

La figure 2a montre d'une manière schématique le porte-aiguille de l'invention avec l'architecture mécanique qui conduit à l'orientation isocentre. Au-dessus du plateau porte-sein 3 on distingue le rappel 19 de la présence du sein ainsi que d'une manière plus précise l'endroit 12 où, avec le dispositif de stéréotaxie 9, on a pu calculer que devait normalement se trouver une tumeur. L'endroit 12 est repéré par des coordonnées X,Y,Z repérées par rapport à un repère 20 solidaire du plateau 3. On pourrait bien entendu choisir n'importe quel autre repère solidaire même d'une autre partie de l'appareil et effectuer des changements d'axes nécessaires. Le porte-aiguille 15 présente la particularité de pointer en permanence en direction de la tumeur 12. Dans ce but, il est fixé à un arbre de rotation 21 dont l'axe passe par la tumeur 12. Dans un exemple, le porte-aiguille 15 est fixé à l'arbre 21 par un arceau 22 circulaire permettant au porte-aiguille 15 d'occuper en site une orientation comprise entre l'orientation verticale (figure 3) et une orientation en contre plongée, passant au moins légèrement sous l'horizontale. Il peut occuper bien entendu toutes les positions intermédiaires entre ces deux positions extrêmes. Pour le faire changer de position, l'arceau 22 peut coulisser entre deux patins tels que 23 et 24 (figure 2a) complètement solidaires de l'arbre 21. Par exemple, au moins un d'entre eux y est soudé. Une poignée 25 de manoeuvre permet, en rapprochant les patins l'un de l'autre de bloquer le coulissement de l'arceau 22. Le porte-aiguille est alors maintenu en site dans une direction donnée.

Les figures 2b à 2g présentent néanmoins une autre solution préférée dans laquelle l'arceau 22 est fixe, le porte-aiguille 15, coulissant sur cet arceau. Cet arceau 22 a une section en forme de té avec deux ailes munies en extrémité supérieure de biseaux 100 et 101. Des galets 102 et 103, figure 2d, montés fous dans le porte-aiguille 15 viennent appuyer sur ces extrémités biseautées. Un autre galet 104 du porte-aiguille 15 à surface de roulement creuse, en forme de diabolo, exerce une réaction sur la base du té de l'arceau. Là aussi, il y a des biseaux. Pour permettre le réglage au moment du montage, l'axe du galet 104 est excentré. En réglant la position d'une excentrique on règle le serrage du chariot porte-aiguille sur l'arceau.

Pour maintenir le chariot sur l'arceau 22, le chariot, qui globalement est placé autour de l'arceau, est muni de deux poignées vissables 105 et 106 permettant de presser des patins 107 et 108 contre les flancs de l'arceau. Les poignées sont maintenues par vissage dans le chariot. Les figures 2f et 2g montrent qu'en plus le sommet du té de l'arceau 22 est muni d'une crémaillère 109. Une roue dentée 110, manoeuvrée par une poignée 111, maintenue elle aussi dans le porte-aiguille 15, s'engrène dans la crémaillère et permet de déplacer ce porte-aiguille. Ce déplacement peut être motorisé. Une vis de serrage 112, figure 2e, manoeuvrée par une poignée 113 permet de serrer un collier 114, solidaire de l'arceau 22 sur l'arbre circulaire 21. On peut ainsi obtenir toutes les orientations désirées en azimut.

Le plateau 16, figure 2a, qui porte le porte-aiguille 15 par l'intermédiaire de l'arbre 21 est de préférence déplaçable dans les trois directions x,y,z. Par exemple, il comporte premièrement un support 26 qui peut coulisser dans des glissières 27 du mât 2 du mammographe par action sur un bouton de réglage 28 dont l'index 281 se déplace en face d'une règle graduée 282. Dans la pratique, le bouton 28, l'index 281 et la règle 282 peuvent être remplacés par des moyens motorisés en relation avec un microprocesseur capable d'interpréter comme altitude z une altitude dépendant de l'altitude Z de la tumeur. On constate déjà qu'avec l'invention les réglages sont très simples, puisqu'il suffit que l'altitude du support 26 soit déduite par addition à l'altitude Z, du rayon de courbure R de l'arceau 22, et de l'altitude relative, fixe par construction, du support 26 par rapport au point d'ancrage de l'arceau 22 entre les patins 23 et 24, déduction faite de l'écart (lui aussi fixe) entre la distance d'enfoncement D et le rayon R.

Le support 26 porte une plaque 29 susceptible de se déplacer en translation dans le sens y sous l'action d'un bouton de commande 30. Dans la pratique le bouton de commande 30 peut également être remplacé par une action motorisée. Cette action dépend de l'ordonnée Y de la tumeur: le déplacement y est égal à Y, au décalage près entre les repères par rapport auxquels ils sont mesurés. Ce décalage peut être aussi rendu fixe par construction, de sorte que la connaissance de Y amène directement, à une constante près, à la valeur du déplacement y. La plaque 29 comporte par ailleurs une autre commande 31 permettant de déplacer l'arbre 21 dans une rainure 32. Là aussi, le déplacement selon la direction x est simple, à une constante près déterminée par construction, x = X. Par ailleurs, si le support 26 devait pouvoir avoir, au départ, une position quelconque par rapport au plateau 3, il conviendrait de repérer, par une échelle absolue solidaire du mât 2, comme par exemple l'échelle 33, des altitudes respectives de départ du support 26 et de la tumeur 12.

De manière à obtenir en azimut toutes les orientations désirées, au moins sur un arc de cercle 34 faisant de préférence plus que 180°, l'arbre 21 est monté par exemple dans un roulement à billes 35. Ce roulement à billes peut par ailleurs être couplé à un frein, non représenté, qui agit sur l'arbre 21 de manière à en figer l'orientation dans une direction choisie. L'arbre 21 est maintenu dans le roulement par exemple par des colliers. Le porte-aiguille 15 porte l'aiguille 14 dont l'extrémité 13 est distante de la distance D de la position de la tumeur. La distance D est une distance propre au porte-aiguille. En conséquence, une fois qu'on provoque l'enfoncement, l'extrémité 13 de l'aiguille 14 se déplace depuis l'extérieur du sein jusqu'à l'endroit de la tumeur 12.

Le dispositif de l'invention présente alors l'avantage de permettre d'enfoncer l'aiguille à partir de n'importe quelle direction. Le choix des directions possibles n'est limité que par les obstacles constitués par la pelote et par le plateau porte-sein. Il est possible de modifier ces derniers pour s'adapter à un besoin particulier. On procède alors de la manière suivante. Au moyen des boutons 28,30 et 31 (ou de leurs équivalents motorisés et contrôlés par un microprocesseur), on règle les positions respectivement du support 26, de la plaque 30, et de l'arbre 21 en déplacement x. On rappelle que les déplacements x y z sont connus puisqu'à des constantes près ils sont égaux à X Y Z. On constate par ailleurs qu'on ne se préoccupe plus de Xₐ Yₐ Zₐ. Une fois ces réglages effectués, on choisit en fonction de raisons alors uniquement anatomiques et indépendantes du mammographe, quelle est la voie d'introduction la plus adéquate. On peut choisir, d'une part une orientation en site en faisant glisser l'arceau 22 entre les patins 23 et 24. Une fois que cette orientation en site a été choisie, on peut choisir une orientation en azimut en faisant tourner l'arbre 21 dans le roulement à billes 35. Lorsque ces positions sont atteintes, on actionne la poignée de manoeuvre 25 et le frein de rotation pour figer la position de l'ensemble. Une fois que cette mise en place est terminée, on provoque l'enfoncement de l'aiguille.

La représentation qui est donnée ici est une représentation schématique et notamment d'autres dispositifs peuvent être prévus, en particulier l'arceau 22 peut être muni d'un contrepoids situé de l'autre côté de cet arceau par rapport à l'endroit où se trouve le porte-aiguille 15. Ceci permet d'avoir des déplacements équilibrés de cet arceau entre les patins 23 et 24.

La figure 3 montre une réalisation dans laquelle on est capable avec le dispositif de l'invention d'obtenir une incidence verticale. Dans ce cas l'arbre 21 comporte un décrochement 36 permettant de définir un faux arbre 37. Les patins 23 et 24 sont fixés au faux arbre 37. Le faux arbre 37 présente un décalage en retrait, dans le plan de rotation de l'arceau 22 entre les patins.

Il est possible selon ce qui a été indiqué précédemment de choisir de ne pas effectuer le prélèvement ou, surtout, la fixation de l'hameçon à l'endroit de la tumeur 12 mais légèrement à côté. Dans ce cas on pourra décider, en fonction du besoin, d'effectuer un décalage en δX,δY ou δZ ou même en une combinaison de ces trois coordonnées, dont le chirurgien devra ultérieurement tenir compte pour effectuer son incision. Le réglage de l'appareil dans ce cas est très simple, il suffit de définir un isocentre fictif situé à un écart δ de son endroit réel. On règle ensuite le porte-aiguille sur cet isocentre fictif.

La figure 4 montre une réalisation simplifiée, utilisée pour mettre en oeuvre l'invention. La structure avec arceau 22 a été remplacée par une structure de manivelle, l'arbre 21 étant l'arbre de la manivelle. La manivelle présente un décrochement 38 et un bras de manivelle 39 auquel est fixé le porte-aiguille 15. Cette réalisation est surtout destinée aux interventions effectuées dans un plan horizontal auquel cas le décrochement 36 présente par rapport à l'arbre 21 et au bras 39 des angles droits.

## Revendications

1. Mammographe comportant
- des moyens (34) pour maintenir un sein (6) d'une patiente entre un dispositif (5) de prise de cliché radiographique et un tube (1) à rayons X ;
- des moyens pour prendre des clichés stéréographiques de ce sein ;
- des moyens (13-15) de biopsie ou de guidage chirurgical pour intervenir dans ce sein en un endroit (12) déterminé ;
- ces moyens de biopsie ou de guidage chirurgical comportant un porte-aiguille (15) déplaçable (26-35) par rapport au sein ;
- ce porte-aiguille comportant une aiguille (14) dont l'extrémité (13) est déplaçable d'une longueur choisie (D) entre une position en retrait et une position enfoncée ;
- un ensemble (9) stéréotaxique en relation avec ces trois moyens pour calculer la position (X,Y,Z) de l'endroit déterminé de l'intervention ;
caractérisé en ce qu'il comporte
- des moyens d'orientation (21-25) pour que le porte-aiguille de biopsie soit orientable autour d'un axe qui passe par un isocentre situé à un endroit du sein déterminé, et pour que le porte-aiguille soit incliné par rapport à cet axe de rotation.

2. Mammographe selon la revendication 1, caractérisé en ce que l'axe de rotation est réalisé en utilisant un arbre de rotation.

3. Mammographe selon la revendication 2, caractérisé en ce qu'un arceau circulaire est porté par l'arbre de rotation pour disposer de deux degrés de liberté en orientation de l'aiguille.

4. Mammographe selon l'une quelconque des revendications 1 à 3, caractérisé en ce que l'ensemble stéréotaxique comporte des moyens (26) pour déterminer une altitude de départ de ce porte-aiguille et des moyens (21, 38, 39) pour que l'enfoncement soit horizontal.

5. Mammographe selon la revendication 1, caractérisé en ce que l'axe de rotation est réalisé en utilisant une manivelle (21).

6. Mammographe selon l'une quelconque des revendications 1 à 5, caractérisé en ce que l'ensemble stéréotaxique comporte des moyens pour déterminer l'endroit de l'intervention et en déduire une orientation dans deux plans du porte-aiguille.

7. Mammographe selon l'une quelconque des revendications 1 à 4, caractérisé en ce qu'il comporte un arceau circulaire dont le centre de courbure est colinéaire avec l'axe d'un arbre qui porte cet arceau.

8. Mammographe selon l'une quelconque des revendications 1 à 7, caractérisé en ce que le porte-aiguille possède un axe d'enfoncement d'aiguille situé dans un même plan que l'axe d'un arbre de rotation qui porte ce porte-aiguille.

## Patentansprüche

1. Mammographiegerät mit
- einer Einrichtung (34) zum Halten einer Brust (6) einer Patientin zwischen einer Vorrichtung (5) zum Erstellen von Röntgenaufnahmen und einer Röntgenröhre (1);
- einer Einrichtung, um Stereoaufnahmen von der Brust zu erstellen;
- einer Einrichtung (13-15) zur Biopsie oder chirurgischen Führung, um in der Brust an einem bestimmten Ort (12) einen Eingriff vorzunehmen;
- wobei die Einrichtung zur Biopsie oder chirurgischen Führung einen Nadelhalter (15) aufweist, der bezüglich der Brust verschiebbar ist (26-35);
- wobei der Nadelhalter eine Nadel (14) aufweist, deren Ende (13) um eine gewählte Strecke (D) zwischen einer zurückgezogenen und einer eingeführten Position verschiebbar ist;
- einer Stereotaxieeinheit (9), die mit den drei Einrichtungen in Beziehung steht, um die Position (X, Y, Z) des bestimmten Ortes für den Eingriff zu berechnen;
dadurch gekennzeichnet, daß es
- eine Ausrichtungseinrichtung (21-25) aufweist, damit der Biopsienadelhalter um eine Achse verschwenkbar ist, die durch ein Isozentrum verläuft, das sich an einem bestimmten Ort der Brust befindet, und damit der Nadelhalter bezüglich dieser Drehachse geneigt ist.

2. Mammographiegerät nach Anspruch 1, dadurch gekennzeichnet, daß die Drehachse unter Verwendung einer Drehwelle realisiert ist.

3. Mammographiegerät nach Anspruch 2, dadurch gekennzeichnet, daß von der Drehwelle ein kreisförmiger Bügel gehalten wird, um für die Ausrichtung der Nadel über zwei Freiheitsgrade zu verfügen.

4. Mammographiegerät nach einem der Ansprüche 1 bis 3, dadurch gekennzeichnet, daß die Stereotaxieeinheit eine Einrichtung (26) zum Bestimmen einer Ausgangshöhe des Nadelhalters und der Einrichtung (21, 38, 39) aufweist, damit das Einführen horizontal geschieht.

5. Mammographiegerät nach Anspruch 1, dadurch gekennzeichnet, daß die Drehachse unter Verwendung einer Kurbel (21) realisiert ist.

6. Mammographiegerät nach einem der Ansprüche 1 bis 5, dadurch gekennzeichnet, daß die Stereotaxieeinheit.eine Einrichtung aufweist, um den Ort des Eingriffs zu bestimmen undtdaraus eine Ausrichtung des Nadelhalters in zwei Ebenen abzuleiten.

7. Mammographiegerät nach einem der Ansprüche 1 bis 4, dadurch gekennzeichnet, daß es einen kreisförmigen Bügel aufweist, dessen Krümmungsmittelpunkt kolinear mit der Achse einer Welle ist, die diesen Bügel trägt.

8. Mammographiegerät nach einem der Ansprüche 1 bis 7, dadurch gekennzeichnet, daß der Nadelhalter eine Nadeleinführungsachse besitzt, die in derselben Ebene wie die Achse einer Drehwelle liegt, die diesen Nadelhalter trägt.

## Claims

1. Mammography apparatus comprising
- means (34) for holding a breast (6) of a patient between a radiography filming device (5) and an X-ray tube (1):
- means for taking stereographic films of the said breast;
- means (13-15) for biopsy or surgical guiding to act at a certain place (12) in the said breast;
- means for biopsy or surgical guiding comprising a needle holder (15) displaceable (26-35) in relation to the breast:
- this needle holder comprising a needle (14) of which the end is displaceable over a selected length (D) between a retracted position and an inserted position:
- a stereotaxic assembly (9) in relation to the said three means and serving to calculate the position (X,Y,Z) of the selected place of action:
characterized by the fact that it comprises
- orientation means (21-25) serving to render the biopsy needle holder adjustable around an axis passing through an iso-centre situated at a certain selected place in the breast and to ensure that the needle holder is inclined in relation to the said rotation axis.

2. Mammograph in accordance with claim 1, characterized by the fact that the rotation axis is provided by the use of a rotation shaft.

3. Mammograph in accordance with claim 2, characterized by the fact that a circular arched piece is borne by the rotation shaft in order to provide two degrees of freedom in the orientation of the needle.

4. Mammograph in accordance with any one of claims 1 to 3, characterized by the fact that the stereotaxic assembly comprises means (26) for determining a starting height for this needle holder and means (21, 38, 39) for ensuring that the direction of insertion is horizontal.

5. Mammograph in accordance with claim 1, characterized by the fact that the rotation axis is provided by the use of a crank (21).

6. Mammograph in accordance with any one of claims 1 to 5, characterized by the fact that the stereotaxic assembly comprises means for determining the location of the action and for deducing therefrom an orientation in two planes of the needle holder.

7. Mammograph in accordance with any one of claims 1 to 4, characterized by the fact that it comprises a circular arched piece of which the centre of curvature is colinear with the axis of a shaft bearing this arched piece.

8. Mammograph in accordance with any one of claims 1 to 7, characterized by the fact that the needle holder has a needle insertion axis situated in a common plane with the axis of rotation shaft bearing this needle holder.
